Europäisches Patentamt

European Patent Office

Office européen des brevets

(11) Veröffentlichungsnummer: **0 283 826**
**A2**

# (12) EUROPÄISCHE PATENTANMELDUNG

(21) Anmeldenummer: **88103549.7**

(22) Anmeldetag: **08.03.88**

(51) Int. Cl.4: **B01D 13/01**

(30) Priorität: **21.03.87 DE 3709432**

(43) Veröffentlichungstag der Anmeldung:
**28.09.88 Patentblatt 88/39**

(84) Benannte Vertragsstaaten:
**CH DE ES FR GB IT LI**

(71) Anmelder: **Fresenius AG**
**Gluckensteinweg 5**
**D-6380 Bad Homburg v.d.H.(DE)**

(72) Erfinder: **Mathieu, Bernd, Dr. Dipl.-Chem.**
**Galgenbergstrasse 11**
**D-6683 Spiesen-Elversberg(DE)**

(74) Vertreter: **Görtz, Dr. Fuchs, Dr. Luderschmidt**
**Patentanwälte**
**Sonnenberger Strasse 100 Postfach 26 26**
**D-6200 Wiesbaden(DE)**

(54) **Kapillarfilteranordnung zur Sterilisation von flüssigen Medien.**

(57) Kapillarfilteranordnung zur Sterilisation von flüssigen Medien mit zwei in Strömungsverbindung stehenden semipermeablen Kapillarfaserbündeln (4 bzw. 5), die sich nebeneinander angeordnet in einem einzigen Gehäuse (1) befinden. Die gegenüberliegenden Öffnungen des Gehäuses (1) sind jeweils durch eine Endkappe (2;2a), die je mindestens eine Anschlußöffnung (3;3a) zur Ein-bzw. Ausleitung des Mediums aufweist, verschlossen. Das Gehäuse (1) weist an seinen Enden jeweils eine Vergußschicht (6;7) auf, in der die Enden der Kapillarfaserbündel (4;5) aufgenommen sind, wobei zwischen Endkappe (2;2a) und Vergußschicht (6;7) jeweils ein Verteilungsraum (8;9) und zwischen den Vergußschichten (6;7) jeweils ein Primärfiltratraum (10) gebildet werden. Die Enden des ersten Kapillarfaserbündels (4) sind gegenüber dem ersten Verteilungsraum (8) und die Enden des zweiten Kapillarfaserbündels (5) gegenüber dem zweiten Verteilungsraum (9) verschlossen, so daß das gesamte Innenlumen des ersten bzw. zweiten Kapillarfaserbündels (4;5) nur mit dem zweiten bzw. ersten Verteilungsraum (9 bzw. 8) in Strömungsverbindung steht.

Fig.1

EP 0 283 826 A2

## Kapillarfilteranordnung zur Sterilisation von flüssigen Medien

Die Erfindung betrifft eine Kapillarfilteranordnung zur Sterilisation von flüssigen Medien mit wenigstens zwei in Strömungsverbindung stehenden semipermeablen Kapillarfaserbündeln.

Die Filtration von Medien, insbesondere medizinischen Flüssigkeiten zur Sterilisation und/oder Entpyrogenisierung wird zur Zeit bei der Bereitung von Substitutionsflüssigkeiten in der Dialysestation bereits angewendet.

Die DE-OS 3444671 beschreibt ein Hämodiafiltrationsgerät mit einem Dialysator, der durch eine Membran in zwei Kammern geteilt ist, wobei die erste Kammer in einen Dialysierflüssigkeitsweg und die zweite Kammer in einen Blutweg geschaltet ist.

Der Dialysierflüssigkeitsweg weist eine Zuleitung, die sich von einer Einrichtung zur Bereitstellung von Dialysierflüssigkeit bis zum Dialysator erstreckt und in die eine erste Bilanzkammer eingeschaltet ist, und eine Ableitung, die sich vom Dialysator zum Abfluß erstreckt und in die eine zweite Bilanzkammer eingeschaltet ist. Weiterhin besitzt diese Einrichtung eine Pumpe zum Fördern der Dialysierflüssigkeit im geschlossenen Dialysierflüssigkeitssystem und eine zwischen den Bilanzkammern im Dialysierflüssigkeitsweg vorgesehene Ultrafiltrationseinrichtung, sowie eine von der Zuleitung zwischen der ersten Bilanzkammer und dem Dialysator abgehende Verbindungsleitung, die mit dem Blutweg verbunden ist und in die wenigstens ein Sterilfilter und eine Pumpe eingeschaltet sind.

Aus Trans. Am. Soc. Artif. Organ (ASAIO), 1978, Seite 465 bis 467, ist die On-Line-Herstellung eines sterilen und pyrogenfreien Substituats bekannt, das dadurch hergestellt wird, daß man die übliche Proportionierungseinheit zur Herstellung der üblichen Dialysierflüssigkeit einsetzt. Dabei wird ein Konzentrat in einem Verhältnis von 1 : 34 mit Wasser vermischt. Die dadurch hergestellte Dialysierflüssigkeit wird anschließend entgast und erwärmt und danach zwei Ultrafiltern zugefördert, die in Reihe geschaltet sind. Am Ende des zweiten Filters wird steriles pyrogenfreies Substituat erhalten, das anschließend dem Blutweg zugeführt wird.

Aus der EP-OS 42939 ist ein Hämofiltrationssystem bekannt, das von der oben erwähnten Einrichtung ausgeht, mit der Maßgabe, daß nur ein Ultrafilter zur Sterilisierung der Dialysierflüssigkeit eingesetzt wird. Dieser Filter muß speziell überwacht werden, um ein Auftreten von Lecks sofort zu erkennen. Diese relativ komplizierte Einrichtung wird bei den vorstehend erwähnten Systemen durch die Anordnung von zwei Filtern beseitigt.

Allen genannten Lösungen ist jedoch der Nachteil gemein, daß die Filter jeweils voneinander getrennte Einheiten bilden. Das bedeutet, daß jeweils zwei Filter in einer Kaskade benützt werden, die nacheinander durchflossen werden. Schwierigkeiten macht dabei das regelmäßige Testen der Integrität der Membranen, das aufwendige Handling bei der Installierung der Filterkaskade und damit verbundene Risiko der Kontamination der Strecke zwischen beiden Filtern. Eine Kontamination dieses Raumes reduziert die Kaskade automatisch wieder auf einen Filter. Auch die nachträgliche Sterilisation des Gesamtsystems stößt auf Schwierigkeiten, da aufgrund der uneinheitlichen Strömungsverhältnisse Keime häufig im Primärfiltrationsraum überleben. Wenn zu Beginn keine Kontamination dieses Raumes vorliegt, werden auch später keine Keime während des Betriebes mehr dort gefunden.

Aus der DE-GM 85 12 777 und der EP-A-76 421 ist eine Kapillarfilteranordnung bekannt, bei der innerhalb eines Gehäuses zwei Kapillarfaserbündel angeordnet sind, die jeweils an ihren Enden in Strömungsverbindung sind, wobei nur ein Gehäuseende mit dem Zulauf-und dem Ablaufstutzen versehen ist. Demzufolge handelt es sich nur um einen zusammengeklappten üblichen Hohlfaserfilter, so daß die vorstehend beim Einsatz eines Filters auftretenden Probleme auch hier vorliegen.

Aufgabe der Erfindung ist es, eine Kapillarfilteranordnung nach dem Oberbegriff des Anspruchs 1 derart weiterzubilden, daß die zu filtrierende Flüssigkeit derart zwei Filtrationsschritten unterzogen wird, daß durch eine Bubble-Point-Prüfung (Anlegen eines Druckgefälles an den Raum zwischen den Kapillarmembranen gegenüber beiden Kapillarinnenräumen bei benetzter Membran und Luftfüllung der jeweils mit dem höheren Druck beaufschlagten Seite) seine Integrität in einfacher Weise getestet werden kann, ohne daß Keime in den Raum zwischen den Kapillarmembranen gelangen können.

Diese Aufgabe wird dadurch gelöst, daß die beiden Kapillarfaserbündel in einem rohrförmigen Gehäuse angeordnet sind und daß die Enden des Gehäuses jeweils eine Vergußschicht aufweisen, in der die Enden der Kapillarfaserbündel aufgenommen sind, und mit jeweils einer wenigstens eine Anschlußöffnung aufweisenden Endkappe verschlossen sind, wobei zwischen Endkappe und Vergußschicht jeweils ein Verteilungsraum und im rohrförmigen Gehäuse zwischen den Vergußschichten ein Primärfiltratraum gebildet werden, und daß die Enden des ersten Kapillarfaserbündels gegenüber dem ersten Verteilungsraum und die Enden des zweiten Kapillarfaserbündels gegenüber dem zweiten Verteilungsraum verschlossen sind, so daß das gesamte Innenlumen des ersten bzw. zweiten Kapillarfaserbündels nur mit dem zweiten bzw. ersten Verteilungsraum in Strömungsverbindung steht.

In dem rohrförmigen Gehäuse der Kapillarfilteranordnung sind zwei in Strömungsverbindung stehende semipermeable Kapillarfaserbündel nebeneinander angeordnet. Die gegenüberliegenden Öffnungen des Gehäuses werden durch jeweils eine Endkappe, die je mindestens eine Anschlußöffnung zur Ein-bzw. Ausleitung des Mediums aufweist, verschlossen. Das Gehäuse besitzt an seinem jeweiligen Ende eine Vergußschicht, in der die Enden der Kapillarfaserbündel aufgenommen sind, so daß zwischen den Vergußschichten und den Endkappen erste und zweite Verteilungsräume gebildet werden. Dabei ist im Bereich der ersten Vergußschicht der Kapillarinnenraum des ersten Kapillarfaserbündels und im Bereich der zweiten Vergußschicht der Kapillarinnenraum des zweiten Kapillarfaserbündels verschlossen. Die Vergußschichten dichten dabei den Raum zwischen den Kapillaren der Kapillarfaserbündel und der Innenoberfläche des Gehäuses im endnahen Bereich ab, wobei zwischen den Außenflächen der Kapillaren und der Gehäuseinnenfläche ein Primärfiltratraum gebildet wird. Dadurch steht das gesamte Innenlumen des ersten bzw. zweiten Kapillarfaserbündels nur mit dem zweiten bzw. ersten Verteilungsraum in Strömungsverbindung.

Gemäß einer bevorzugten Ausführungsform besitzt das Gehäuse mindestens eine Auslaßöffnung zum Abführen des Filtratmediums, welche den Primärfiltratraum mit dem Außenraum verbindet.

Mindestens eine der Anschlußöffnungen besitzt vorteilhafterweise einen Stutzen mit vorzugsweise Luer-Lock-Anschluß, wobei der Stutzen oder eine der Auslaßöffnungen vorzugsweise mit einem hydrophoben, mikroporösen Sterilfilter verschlossen ist.

Vorteilhafterweise kann die Membran zumindestens eines der Kapillarfaserbündel Adsorptivstoffe aufweisen. Zur Erhöhung der Filtrationswirkung ist mindestens eine zusätzliche Filtrationseinrichtung vorhanden, die im Primärfiltratraum zwischen den Kapillarfaserbündeln angeordnet ist.

Diese zusätzliche Filtrationseinrichtung besteht vorzugsweise aus einem semipermeablen - schlauchförmigen Stoff, der wenigstens eines der Kapillarfaserbündel umschließt. Vorteilhafterweise weist auch diese zusätzliche Filtrationsvorrichtung Adsorptivstoffe auf. Diese Adsorptivstoffe haben positiv geladene Oberflächengruppen, vorzugsweise quartäre Ammoniumgruppen.

Die Herstellung eines Kapillarfilters kann nach folgenden Schritten durchgeführt werden :

- Einbringen zweier räumlich getrennter Faserbündel in ein gemeinsames rohrförmiges Gehäuseteil,

- Vergießen der Endbereiche des Gehäuses mit einer flüssigen Dichtmasse, die aufgrund ihrer Viskosität nicht vollständig in den Kapillarinnenraum eindringt und anschließend aushärtet,

- Öffnen der Kapillarinnenlumina durch Abtrennen eines Teils der ausgehärteten Dichtmassenblöcke,

- Verschließen je eines Endbereiches der beiden Kapillarbündel auf gegenüberliegenden Seiten mit einem Dichtmittel,

- Aufbringen der Endkappen auf das Gehäuse.

Eine andere Art der Herstellung eines Kapillarfilters kann durch folgende Verfahrensschritte erfolgen :

- Einbringen zweier räumlich getrennter Faserbündel in das gemeinsame Gehäuseteil, gegebenenfalls mit Hilfe einer mit einer Vielzahl von Öffnungen versehenen Trennwand, deren Ende jeweils in der Vergußmasse verankert wird,

- Verschließen des Innenlumens jeweils eines Endbereiches auf gegenüberliegenden Seiten der beiden Faserbündel mit einem Dichtmittel,

- Einbringen einer flüssigen Dichtmasse, die den Raum zwischen der äußeren Oberfläche der Kapillaren und der inneren Oberfläche des Gehäuses in den endnahen Bereichen ausfüllt und die auf der jeweils unverschlossenen Seite in die Faserinnenräume eindringt,

- Aushärten der Dichtmasse,

- Öffnen der Kapillarinnenlumina auf den den zuerst verschlossenen Endbereichen gegenüberliegenden Seiten durch Abtrennen eines Teils der ausgehärteten Dichtmassenblöcke,

- Aufbringen der Endkappen auf das Gehäuse.

Schließlich kann ein Verfahren zur Herstellung eines Kapillarfilters durch folgende Schritte gekennzeichnet sein:

- Verschließen des Innenlumens jeweils eines Endberei ches auf gegenüberliegenden Seiten der Faserbündel mit einem Dichtmittel,

- Einbringen der beiden Faserbündel in das gemeinsame Gehäuseteil, wobei die jeweils offenen Kapillarenden gegenüberliegen,

- Einbringen einer flüssigen Dichtmasse, die die Zwischenräume zwischen Kapillaraußenfläche und Gehäuseinnenfläche in den beiden endnahen Bereichen ausfüllt und die auf der jeweils unverschlossenen Seite in die Faserinnenräume eindringt,

- Aushärten der Dichtmasse,

- Öffnen der Kapillarinnenlumina auf den den zuerst verschlossenen Endbereichen gegenüberliegenden Seiten durch Abtrennen eines Teils der ausgehärteten Dichtmassenblöcke und

- Aufbringen der Endkappen auf das Gehäuse.

Durch die erfindungsgemäße Lösung wird es erlaubt, die zu filtrierende Flüssigkeit in einem Gehäuse zwei Filtrationsschritten zu unterziehen, wobei die Strömungsrichtung bei der ersten Kapillarmembran von innen nach außen, bei der zweiten von außen nach innen verläuft.

Durch den erfindungsgemäßen Aufbau des Kapillarfilters ist es möglich, durch eine einzige Bubble-Point-Prüfung die Integrität beider Filtrationsstufen zu testen. Dazu wird zuerst ein Überdruck mittels Luft durch einen Anschlußstutzen an den Primärfiltrationsraum gegenüber beiden Kapillarinnenräumen bei benetzter Membran angelegt. Ist die Oberfläche eines der Kapillarfaserbündel gerissen oder auf andere Weise beschädigt, treten an den Auslaßöffnungen der Faserlumina Luftblasen aus, die in der jeweils im Verteilungsraum befindlichen Flüssigkeit erkannt werden können. Ist die Oberfläche der Kapillarfaserbündel jedoch nicht rupturiert, wird der Druck im Gehäuse des Kapillarfilters eine Zeitlang gehalten, da die benetzten Kapillarmembranen als Gassperre dienen. Andererseits kann auch der Primärfiltratraum mit Flüssigkeit gefüllt sein, wobei die Luft durch den jeweiligen Verteilungsraum dem Lumen des jeweiligen Kapillarfaserbündels zugeführt wird. Hier erfolgt bei Ruptur einer Membran der Luftaustritt in den Primärfiltratraum.

Durch die Verringerung des Volumens des Primärfiltrationsraumes des Doppelfilters gegenüber der Kaskadierung zweier vollständiger Filter wird infolge der bes rung zweier vollständiger Filter wird infolge der besseren Flußbedingungen die Resterilisation erleichtert.

Durch die neue Lösung wird die bei der Inbetriebnahme mögliche, nachträgliche Kontamination des Primärfiltrationsraumes verhindert. Damit wird das in der Praxis beobachtete Wachstum von Keimen zwischen den beiden Filtrationsstufen ausgeschlossen. Von Vorteil ist weiterhin, daß die Produktionskosten drastisch reduziert werden können durch Verwendung eines konventionellen Filtergehäuses.

Zusätzlich zu den beschriebenen Vorteilen kann in dem System ohne Schwierigkeiten eine dritte Filtrationsstufe, z.B. in Form eines Tiefenfilters zwischen die beiden Membranstufen integriert werden. Dieses Tiefenfilter kann ebenso mit spezifischen Adsorptionsgruppen ausgerüstet sein, beispielsweise positive Oberflächengruppen, wie quartäre Ammoniumgruppen als Adsorbens für Pyrogene.

Als Membranen für die beiden Hohlfaserbündel können die üblichen Membranen eingesetzt werden, die bei der Dialyse, Hämofiltration oder Sterilfiltration unabhängig, ob für industrielle oder für Heilzwecke benutzt werden. Zu Membranmaterialien gehören beispielsweise Zellulosederivade, Polyamid, Polysulfon u.dgl..

Derartige Membranen haben üblicherweise eine asymmetrische Struktur, weisen somit eine Filterschicht auf, die sich integral auf einer großporigen Stützschicht befindet.

Die Porengrößen dieser Filterschicht werden entsprechend dem Einsatzzweck der Membranen gewählt und sollten für die Sterilfiltration höchstens bei 0,5, insbesondere bei etwa 0,25 $\mu$m liegen. Dabei kann die mittlere Porengröße zwischen 5 bis 10 nm und 0,5 $\mu$m liegen. Wegen der hydraulischen Eigenschaften wird man jedoch üblicherweise eine Membran wählen, deren mittleren Porengröße zwischen 0,1 bis 0,5, insbesondere bei etwa 0,25 $\mu$m liegt.

Auch die Stärke der Membran und der innere Durchmesser des Hohlfadens sind an sich unkritisch. So kann die Stärke der Membran zwischen 10 bis 100 $\mu$m, insbesondere bei etwa 30 bis 60 $\mu$m liegen, während der Innendurchmesser der Hohlfaser bei 100 bis 500 $\mu$m, insbesondere bei etwa 150 bis 300 $\mu$m liegt.

Desgleichen werden vorteilhafterweise solche asymmetrische Hohlfasern eingesetzt, bei denen die Innenseite des Fadens die Trennmembran aufweist, während die Außenseite mit der - schwammartigen Stützstruktur versehen ist.

Die beiden Kapillarfaserbündel können in ihren Membraneigenschaften bzw. -größenverhältnissen entweder gleich oder verschieden sein, was üblicherweise durch den Einsatzzweck vorgegeben wird.

Im übrigen lassen sich auch neben den üblicherweise hydrophilen Membranen auch hydrophobe Membranen einsetzen, die nur unter Zuhilfenahme von Hydrophilierungsmitteln mit Wasser benetzbar sind. Zu derartigen Membranen gehören beispielsweise Polysulfon-und Polyamidmembranen, die beispielsweise durch hydrophile in das Membranmaterial eingearbeitete Zusätze, wie Polyvinylpyrrolidon, mit Wasser benetzbar gemacht werden können.

Neben den vorstehend beschriebenen Massenaustauschmembranen, bei denen ein Massenaustausch in einer Flüssigkeit zustande kommt, können auch Gasdiffusionsmembranen eingesetzt werden, die lediglich den Durchtritt von Gasen, nicht jedoch von Flüssigkeiten gewähren. Derartige Membranen sind vorteilhafterweise die vorstehend beschriebenen hydrophoben Membranen. Mit solchen Membranen können beispielsweise Reaktoren steril mit Sauerstoff begast werden, wobei das sich bildende Kohlendioxid in Verbindung mit überschüssigem, zugeführtem Gas durch das andere Kapillarfaserbündel abgeführt wird.

Die Kapillarfaserbündel werden nach den üblichen Verfahren an den beiderseitigen Enden eines Gehäuses durch Eingießen mit einem Vergußmaterial befestigt. Dabei können die üblichen aushärtbaren Vergußmassen, wie Polyurethane, Silikone, Epoxidharze, Reaktivharze u.dgl. zum Einsatz kommen.

Die erfindungsgemäße Anordnung ist nicht nur auf den Einsatz von zwei Kapillarfaserbündeln innerhalb des Gehäuses beschränkt. Vielmehr können entsprechend dem Einsatzzweck drei oder mehrere Kapillarfaserbündel eingesetzt werden, die entweder zur Zufuhr oder zur Abfuhr von unterschiedlichen Medien eingesetzt werden können. So ist es denkbar, daß für die Zufuhr von Gasen und von Flüssigkeiten je ein unterschiedliches Kapillarfaserbündel eingesetzt wird, die vorteilhafterweise über eine separate Zuführkammer verfügen, so daß keine vorzeitige Mischung dieser Medien stattfindet. Eine ähnliche Anordnung kann auf der Abfuhrseite vorliegen.

Die erfindungsgemäßen Austauschelemente können - wie bereits eingangs erwähnt - für die Hämodialyse oder die Hämofiltration bei der Sterilisierung von wässrigen Medien, insbesondere von Dialysierflüssigkeiten eingesetzt werden. Andererseits lassen sich die erfindungsgemäßen Anordnungen auch für andere industrielle Zwecke einsetzen, bei denen die Sterilität des in der Anordnung befindlichen Mediums aufrechterhalten werden muß. Dies gilt beispielsweise bei der mikrobiologischen Herstellung von bestimmten Substanzen, insbesondere Arzneistoffen. So lassen sich Bakterien-oder Zellkulturen innerhalb dieser Anordnung unter sterilen Bedingungen sowohl mit Gas als auch mit flüssigen Nährmedien durch die Membranen hindurch versorgen, während auf der anderen Seite das von der Kultur hergestellte Produkt abgezogen werden kann.

Die vorliegende Erfindung soll anhand von Ausführungsbeispielen näher erläutert werden.

Dabei zeigt beiliegende Zeichnung in

- Fig. 1 einen Längsschnitt durch den erfindungsgemäßen Kapillarfilter,

- Fig. 2 einen Querschnitt in Höhe I - I des Kapillarfilters mit um 90 Grad versetzter Auslaßöffnung und

- Fig. 3 einen Schnitt in Höhe I - I durch den Kapillarfilter mit um 90 Grad versetzter Auslaßöffnung und zusätzlicher Filtrationsvorrichtung.

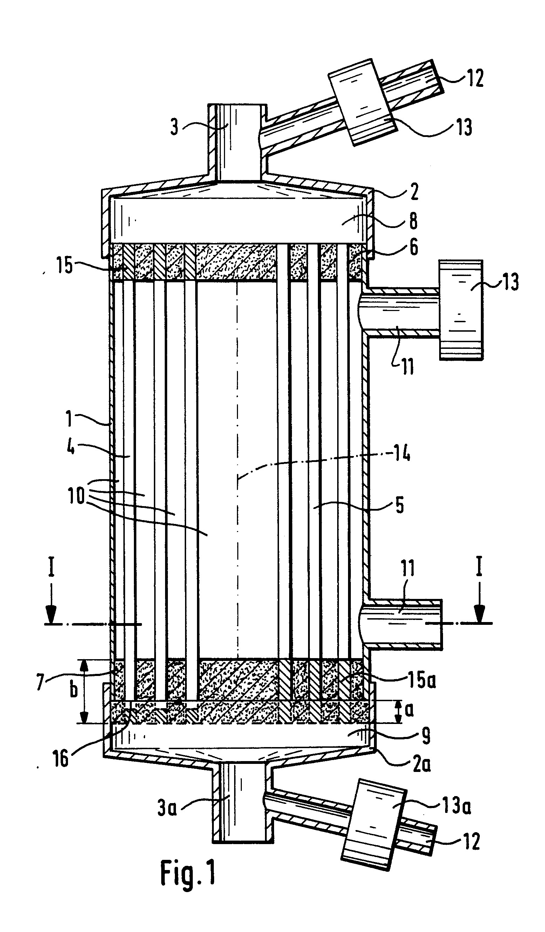

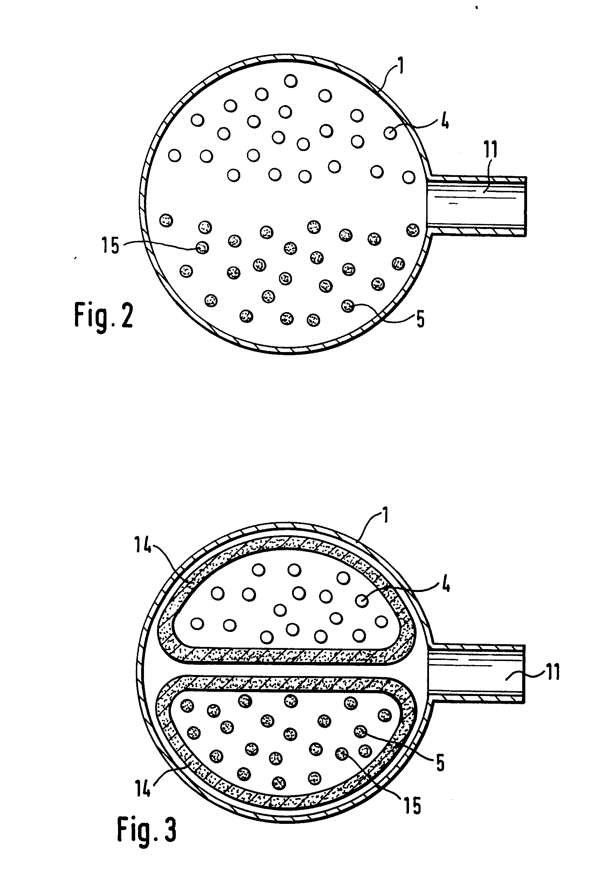

Gemäß Fig. 1 und 2 besitzt die Kapillarfilteranordnung einen Gehäusekörper 1, der beispielsweise aus einem zylindrischen Rohr besteht. Zwei Endkappen 2 ;2a verschließen die zwei gegenüberliegenden Öffnungen dieses Gehäusekörpers 1, wobei die Endkappen 2 ;2a je mindestens eine Anschlußöffnung 3 ;3a zur Ein- bzw. Ausleitung der zu filtrierenden Flüssigkeit bzw. des Filtrats aufweisen.

Zwei Kapillarfaserbündel 4 und 5 befinden sich nebeneinander angebracht im rohrförmigen Gehäuse 1. Das Gehäuse 1 besitzt an seinem jeweiligen Ende eine erste und zweite Vergußschicht 6; 7, in der jeweils die Enden des ersten und zweiten Kapillarfaserbündels 4 und 5 derart aufgenommen sind, daß in der ersten bzw. zweiten Vergußschicht 6 bzw. 7 das Kapillarlumen des zweiten bzw. ersten Kapillarfaserbündels 5 bzw. 4 geöffnet ist und mit dem Verteilungsraum 8 bzw. 9 in Strömungsverbindung steht und in der ersten bzw. zweiten Vergußschicht 6 bzw. 7 das Kapillarlumen des ersten bzw. zweiten Kapillarfaserbündels 4 bzw. 5 mit einer Sperrschicht 15;15a verschlossen ist. Durch die Außenflächen der Kapillaren und der Gehäuseinnenfläche wird zwischen den Vergußschichten 6 und 7 ein Primärfiltratraum 10 gebildet. Das Gehäuse 1 besitzt mindestens eine Auslaßöffnung 11, die den Primärfiltratraum 10 mit dem Außenraum verbindet.

Wenigstens eine der Anschlußöffnungen 3;3 a besitzt einen Stutzen 12 mit vorzugsweise Luer-Lock-Anschluß und ein hydrophobes, mikroporöses Sterilfilter 13, 13 a, das eine mittlere Porengröße von etwa 0,2 um aufweist.

Andererseits kann auch das Sterilfilter 13 bzw. 13a, wie aus Fig. 1 ersichtlich ist, die Auslaßöffnung 11 oder, wie erwähnt, zumindest eine der Anschlußöffnungen 3; 3a verschließen. Erfindungswesentlich ist dabei nur, daß das zur Bubble-Point-Prüfung notwendige Gas unter sterilen Bedingungen dem Filter zugeführt wird und daß nach der Prüfung nur das Gas und nicht die Flüssigkeit aus dem Filter verdrängt wird, was durch die hydrophoben Eigenschaften des Sterilfilters sichergestellt wird.

Zur Erhöhung der Filtrationswirkung weist zumindestens eines der beiden Kapillarfaserbündel Adsorptivstoffe an seiner Membran auf.

Die Kapillarfilteranordnung gemäß Fig. 3 besitzt mindestens eine zusätzliche Filtrationseinrichtung 14, die im Primärfiltratraum zwischen den Kapillarfaserbündeln 4 und 5 angeordnet ist. Diese umschließt wenigstens eines der Kapillarfaserbündel und besteht vorzugsweise aus einem semipermeablen schlauchförmigen Stoff, der die gleiche Trenngrenze wie die Kapillaren aufweisen kann. Dieser semipermeable schlauchförmige Stoff ist ebenfalls an seinen beiden Enden in den Vergußschichten 6 und 7 verankert und kann durch wenigstens eine Vergußschicht und eine Endkappe hindurch mittels eines nicht gezeigten Zuführ-oder Abführstutzens mit der Umgebung in Strömungsverbindung sein. Dieser nicht gezeigte Stutzen kann dabei ähnlich wie der Stutzen 11 ausgebildet und mit einem mikroporösen, hydrophoben Filter 13 abgeschlossen sein. Auch diese zusätzliche Filtrationseinrichtung 14 kann vorteilhafterweise Adsorptivstoffe enthalten. Diese Adsorptivstoffe weisen positiv geladene Oberflächengruppen auf, die vorzugsweise quartäre Ammoniumgruppen als Adsorbens für Pyrogene sind.

Die Kapillarfaserbündel 4 und 5 bestehen aus Kapillarmembranen, deren Porengröße in einem Bereich von etwa 0,1 ... 0,5 μm, insbesondere etwa 0,25 μm liegt.

Das zu filtrierende Medium wird beispielsweise über den Anschlußstutzen 3 dem Verteilungsraum 8 zugeführt Das Medium tritt nun in das nicht versperrte Kapillarfaserbündel 5 ein. Nun erfolgt der erste Filtrationsschritt, wobei die Strömungsrichtung der eingetretenen Flüssigkeit in der ersten Kapillarmembran von innen nach außen in den Primärfiltratraum 10 verläuft. Im zweiten Filtrationsschritt, der lediglich eine Sicherheitsstufe darstellen soll, tritt nun das Medium von außen nach innen aus dem Primärfiltratraum 10 in das Kapillarfaserbündel 4 ein. Die Ableitung der filtrierten Flüssigkeit erfolgt nun über den anderen Anschlußstutzen 3 bzw. die Auslaßöffnungen 11. Erfolgt die Zuführung des zu filtrierenden Mediums über den anderen Anschlußstutzen 3a, verläuft die Filtration in umgekehrter Richtung.

Diese Kapillarfilteranordnung wird wie nachstehend erläutert, auf folgende Weise desinfiziert, gespült, geprüft und entlüftet:

Basis für die Prüfung auf Lecks bildet der Bubble-Point-Test. Es wird davon ausgegangen, daß zuvor dialysiert und mit Wasser bzw. wässriger Lösung gespült wurde. Beim erstmaligen Aufbau der Kapillarfaserbündel 4 und 5 muß mit dem Vorgang Entlüften begonnen werden, wobei die Luft durch Desinfektionsmittel verdrängt wird. Das Kapillarfilter wird dabei gemäß dem Desinfektionsprogramm desinfiziert, wobei eine Substituatpumpe mit einer bestimmten Rate, beispielsweise etwa 200 ml pro Minute Desinfektionsmittel in den Filter fördert. Dabei wird Desinfektionsmittellösung vom ersten Kapillarfaserbündel 5 in das zweite Kapillarfaserbündel 4 gedrückt und über die Anschlußöffnung 3a abgeleitet. Anschließend wird so lange mit Frischwasser gespült, bis die Desinfektionsmittellösung sicher aus dem gesamten Filter durch das Frischwasser ersetzt worden ist.

Danach wird vorzugsweise über das hydrophobe, mikroporöse Sterilfilter 13 Luft in das erste Kapillarfaserbündel 5 gepresst. Da die Membran des Kapillarfaserbündels 5 mit Wasser benetzt ist, kann die eingeströmte Luft nicht über das benetzte Filter entweichen, so daß sich hierdurch die Möglichkeit ergibt, etwaige in der Membran vorliegende Risse oder Löcher (pinholes) mit Hilfe des Überdruckes zu überprüfen. Bei intakter Filtermembram wird nach Erreichen eines Transmembrandruckes von etwa 500 mm Hg eine bestimmte Zeit, beispielsweise 2 Minuten, gewartet. Anschließend wird der Druck an dem Druckmeßgerät beobachtet und die Zeit gestoppt, die der Druck braucht, um auf einen Druck von etwa 500 auf 400 mm Hg sich zu verändern. Sollte diese Zeit größer als etwa 1 Minute sein, so werden die Kapillarfaserbündel 4 und 5 als dicht angesehen. Die Prüfung des Kapillarfaserbündels 4 erfolgt über die andere Anschlußöffnung 3a, an der sich ebenfalls ein hydrophobes, mikroporöses Sterilfilter 13 befindet. Falls die Zeit kleiner sein sollte als 1 Minute, so ist entweder eines oder beide Kapillarfaserbündel undicht.

Alternativ kann diese Prüfung auch über ein an einer Auslaßöffnung 11 angeordnetes Sterilfilter 13 erfolgen, wobei beide Kapillarfaserbündel 4 und 5 gleichzeitig geprüft werden. Falls in einem dieser Bündel ein Leck vorliegt, so perlen Luftblasen aus den offenen Enden der Kapillarfasern in einen der Verteilungsräume 8 oder 9.

Nach der Prüfstufe erfolgt die Entlüftung, d.h. die Füllung des gesamten Systems wieder mit wässriger Dialysierflüssigkeit, wobei die Luft durch das hydrophobe Sterilfilter 13 verdrängt wird, das anschließend die wässrige Flüssigkeit nicht durchtreten läßt.

Das erfindungsgemäße Kapillarfilter kann in einer beispielsweisen Ausführungform nach folgendem Verfahren hergestellt werden: (lediglich im oberen Teil der Fig. 1 dargestellt)

Die zwei räumlich getrennten Kapillarfaserbündel werden in das gemeinsame Gehäuse eingebracht und die Endbereiche des Gehäuses mit einer nachträglich aushärtenden Dichtmasse vergossen. Das Kapillarinnenlumen wird durch Abtrennen eines Teils der ausgehärteten Vergußschicht geöffnet und danach erfolgt das Verschließen je eines Endbereiches der beiden Kapillarfaserbündel auf den gegenüberliegenden Seiten mit einem aushärtenden Dichtmittel (z.B. Polyurethanlösung). Schließlich werden die Endkappen dicht aufgebracht.

In einem weiteren Herstellungsverfahren (lediglich im unteren Teil der Fig. 1 dargestellt) werden die zwei räumlich getrennten Kapillarfaserbündel in das gemeinsame Gehäuse eingebracht und die gegenüberliegenden Endbereiche der beiden Kapillarfaserbündel mit nachträglich aushärtender Dichtmasse verschlossen. Die Endbereiche können jedoch aber auch zuvor mit dem vorstehend genannten Dichtmittel verschlossen und danach in das Gehäuse eingebracht werden.

Nun wird eine nachträglich aushärtende Dichtmasse eingebracht, die den Raum zwischen der äußeren Oberfläche der Kapillaren und der Innenoberfläche des Gehäuses in den endnahen Bereichen über die Höhe b ausfüllt und der auf der jeweils unverschlossenen Seite in die Faserinnenräume eindringt. Nach dem Aushärten der Dichtmasse wird der Kapillarinnenlumen durch Abtrennung eines Teils der Vergußschicht geöffnet und die Endkappen aufgebracht.

Die Eindringhöhe der Dichtmasse ist in Fig. 1 mit a bezeichnet. Die Linie (hier mit 16 bezeichnet) des abgetrennten Teils der Vergußschicht 7 liegt zwischen der oberen Höhenbegrenzung von a und der oberen Höhenbegrenzung b, wobei der abgetrennte Teil strichliert dargestellt ist.

## Ansprüche

1. Kapillarfilteranordnung zur Sterilisation von flüssigen Medien mit zwei in Strömungsverbindung stehenden semipermeablen Kapillarfaserbündeln, dadurch gekennzeichnet, daß die beiden Kapillarfaserbündel (4;5) in einem rohrförmigen Gehäuse (1) angeordnet sind und daß die Enden des Gehäuses (1) jeweils eine Vergußschicht (6;7) aufweisen, in der die Enden der Kapillarfaserbündel (4;5) aufgenommen sind, und mit jeweils einer wenigstens eine Anschlußöffnung (3;3a) aufweisenden Endkappe (2;2a) verschlossen sind, wobei zwischen Endkappe (2;2a) und Vergußschicht (6;7) jeweils ein Verteilungsraum (8;9) und im rohrförmigen Gehäuse (1) zwischen den Vergußschichten (6;7) ein Primärfiltratraum (10) gebildet werden, und daß die Enden des ersten Kapillarfaserbündels (4) gegenüber dem ersten Verteilungsraum (8) und die Enden des zweiten Kapillarfaserbündels (5) gegenüber dem zweiten Verteilungsraum (9) verschlossen sind, so daß das gesamte Innenlumen des ersten bzw. zweiten Kapillarfaserbündels (4 bzw. 5) nur mit dem zweiten bzw. ersten Verteilungsraum (9 bzw. 8) in Strömungsverbindung steht.

2. Kapillarfilteranordnung nach Anspruch 1, dadurch gekennzeichnet, daß das Gehäuse (1) mindestens eine Auslaßöffnung (11) besitzt, die den Primärfiltratraum (10) mit dem Außenraum verbindet.

3. Kapillarfilteranordnung nach den Ansprüchen 1 und 2, dadurch gekennzeichnet, daß mindestens eine der Anschlußöffnungen (3, 3a) einen Stutzen (12) mit vorzugsweise Luer-Lock-Anschluß und daß der Stutzen (12) oder eine der Auslaßöffnungen (11) eine lydrophobes mikroporöses Sterilfilter (13) aufweist.

4. Kapillarfilteranordnung nach den Ansprüchen 1 bis 3, dadurch gekennzeichnet, daß die Membran zumindest eines der beiden Kapillarfaserbündel (4 bzw. 5) Adsorptivstoffe enthält.

5. Kapillarfilteranordnung nach den Ansprüchen 1 bis 4, dadurch gekennzeichnet, daß zumindestens eine zusätzliche Filtrationsvorrichtung (14) vorhanden ist, die in Strömungsrichtung zwischen den Kapillarfaserbündeln (4 und 5) angeordnet ist.

6. Kapillarfilteranordnung nach Anspruch 5, dadurch gekennzeichnet, daß die zusätzliche Filtrationsvorrichtung (14) vorzugsweise aus einem semipermeablen schlauchförmigen Stoff besteht, der wenigstens eines der Kapillarfaserbündel (4 bzw. 5) umschließt.

7. Kapillarfilteranordnung nach den Ansprüchen 5 und 6, dadurch gekennzeichnet, daß die zusätzliche Filtrationsvorrichtung (14) Adsorptivstoffe enthält.

8. Kapillarfilteranordnung nach Anspruch 7, dadurch gekennzeichnet, daß die Adsorptivstoffe positiv geladene Oberflächengruppen aufweisen.

9. Kapillarfilteranordnung nach Anspruch 8, dadurch gekennzeichnet, daß die positiv geladenen Oberflächengruppen vorzugsweise quartäre Ammoniumgruppen sind.

10.Verfahren zur Herstellung einer Kapillarfilteranordnung nach den Ansprüchen 1 bis 9, dadurch gekennzeichnet, daß zwei räumlich getrennte Kapillarfaserbündel in ein Gehäuse eingebracht und die Endbereiche des Gehäuses mit einer nachträglich aushärtenden Dichtmasse vergossen werden, daß das Kapillarinnenlumen beider Kapillarfaserbündel durch Abtrennen eines Teils der ausgehärteten Vergußschicht nach außen geöffnet wird, danach je ein Endbereich der Kapillarfaserbündel auf gegenüberliegenden Seiten mit Dichtmasse verschlossen und anschließend die Endkappen aufgebracht werden.

11.Verfahren zur Herstellung einer Kapillarfilteranordnung nach den Ansprüchen 1 bis 9, dadurch gekennzeichnet, daß die Kapillarfaserinnenlumina jeweils ·eines gegenüberliegenden Endbereiches der beiden Kapillarfaserbündel vor oder nach dem Einbringen der zwei räumlich getrennten Kapillarfaserbündel in das gemeinsame Gehäuse mit einer Dichtmasse verschlossen werden, daß eine nachträglich aushärtende Dichtmasse, die den Raum zwischen der äußeren Oberfläche der Kapillaren und der Innenoberfläche des Gehäuses in den endnahen Bereichen ausfüllt und die auf der jeweils unverschlossenen Seite der Kapillarfaserbündel in die Faserinnenräume eindringt, eingebracht wird, daß nach dem Aushärten der Dichtmasse das Kapillarinnenlumen auf den den zuerst verschlossenen Endbereichen gegenüberliegenden Seiten durch Abtrennen eines Teils der Vergußschicht geöffnet wird und anschließend die Endkappen aufgebracht werden.

12. Verfahren nach den Ansprüchen 10 und 11, dadurch gekennzeichnet, daß die Dichtmasse eine flüssige Polyurethanmasse ist, die nachträglich aushärtet.

Fig.1

Fig. 2

Fig. 3